# EUROPEAN PATENT SPECIFICATION

(11) **EP 1 587 461 B1**
(45) Date of publication and mention of the grant of the patent: **09.04.2008**
(21) Application number: 04702362.7
(22) Date of filing: 15.01.2004
(51) Int. Cl.: A61F 2/44, A61F 2/46

(54) **ARTICULATING SPINAL DISC PROSTHESIS**
GELENKIGE WIRBELSCHEIBENPROTHESE
PROTHESE ARTICULEE DE DISQUE INTERVERTEBRAL

(30) Priority: 17.01.2003 GB 0301085
(43) Date of publication of application: 26.10.2005
(73) Proprietor: Disc Motion Technologies Inc., Boca Raton, FL 33431 (US)
(72) Inventor: KRISHNA, Manoj, Yarm, TS15 9EP (GB); FRIESEM, Tai, Ingleby Barwick TS17 0TN (GB)
(74) Representative: Tomkinson, Alexandra
(86) International application number: PCT/GB2004/000082
(87) International publication number: WO 2004/064692

(56) References cited:
- WO-A-00/23015
- US-A- 5 425 773
- US-A- 5 888 226
- US-A- 6 039 763

## Description

### FIELD OF THE INVENTION

The present invention relates to a prosthetic spinal disc, and more specifically, to a prosthetic spinal disc which articulates, permitting translational and rotational motion.

### BACKGROUND OF THE INVENTION

In the treatment of spinal disorders, especially those affecting spinal disc tissue, it has long been known to remove some or all of a degenerated, damaged or otherwise failing disc.

Corrective measures taken to ensure the correct spacing of the vertebrate formerly separated by the removed or damaged disc can include fusion or, where it is desirous to maintain spinal movement and articulation, spinal disc replacement using disc prostheses.

In spinal fusion the vertebrae above and below the damaged disc or disc space are fused together using transplanted bone tissue, other artificial fusion elements or by mechanical means. Spinal fusion however prevents the fused vertebrae from moving with respect to each other and this is causing increasing concern in the spinal medical community, in that the biomechanical rigidity of the fused vertebrae may lead to rapid deterioration of the adjacent spinal motion segments. Consequently, many patients may require additional disc removal and/or fusion procedures due to further degeneration being promoted by the initial spinal fusion.

Alternatives to fusing a spinal motion segment therefore clearly have inherent advantages.

In spinal disc replacement procedures, prosthetic intervertebral discs have long been used as a substitute for chronically injured or ruptured intervertebral discs in the spinal columns of humans. Such devices employ one, two, three or more individual elements having a wide range of constructions including ball and socket joints, gel-filled enclosures, spring-biased plates and/or the like.

It is becoming increasingly recognised that in order to maintain a range of motion between adjacent vertebrae it is necessary to provide prosthetic spinal discs that will articulate, so that spinal stability is maintained and pain, associated with normal spinal movement, is reduced.

Several different types of articulating intervertebral disc arthroplasty devices have been proposed. For example, US patent 5,556,431 discloses a three-piece prosthesis in which two plates, each having a hollow on one side, are fixed to adjacent vertebrae so that the two hollows face each other across the disc space. The third element is a core having a rounded surface on each side that fits into the hollows in the two plates. Similarly, US patent 5,674,296 discloses a prosthesis having two L-shaped members, each having curved sections between which a resilient disc body is placed.

US patent 5,755,796 discloses a prosthesis for implantation in the disc space between adjacent vertebrae. The prosthesis has two elements, one being attached to the vertebra above the disc space and the other being attached to the vertebra below the disc space. The lower element includes a hollow box frame with a spherical seat and the upper element includes a spherically-shaped head that fits into the spherical seat.

US Patent 5,782,832 discloses an implant having two plate members anchored to adjacent vertebrae within the disc space, the upper plate member having a rounded convex underside and the lower plate member having a concave top side to engage the underside of the upper plate member to allow rotation.

In US Patent 5683465 the plate members have a snap-fit ball and socket engagement that allows rotation.

Other prosthetic disc designs which allow articulation in a rotational sense are also known. They are generally designed to approximate the articulation and behaviour of a normal spinal disc.

More recent observation has confirmed that the normal movement of vertebrae with respect to one another involves not only variable rotation but also translation. These components of the movement are dictated by the size and shape of the individual vertebrae including the relative positions of the vertebral joints (in the cervical spine), the orientation of the facet joints and ligamentous constraints. Essentially, the axis of rotation for any two adjacent vertebrae is unique and during flexion or extension will shift, or translate in varying ways and to varying degrees.

The foregoing prior art describes prostheses or joints that dictate a fixed axis of rotation and will not permit translation. Normal movement will be restricted therefore and the joints may tend to jam, with possible excessive stresses causing wear and provoking pain. Other devices are known which more closely replicate the characteristics and performance of the spinal discs in separating and enabling movement of adjacent vertebrae with respect to each other.

A recent US Patent Application 98/22436, also published as WO 0023015 (27.04.00), discloses an intervertebral joint prosthesis having a ball component for engagement with a first vertebra and a trough component for engagement with a second vertebra. The trough component is described as including "a generally concave surface having a substantially flat portion". When the ball component and the trough component are engaged to their respective vertebrae, the ball component and the trough component engage each other, permitting rotation and translation of the vertebrae with respect to each other. Both components include a flange for engaging with adjacent vertebrae.

The design of this prosthesis allows anteroposterior translation and rotation, and thereby facilitates articulation of the disc. The translation movement, which is achieved by the inclusion of what is described as "a substantially flat portion" in the generally concave surface of the trough component is effectively unrestrained in so far as the convex portion of the ball component is allowed to freely translate along the concavity of the trough component. This free translation is promoted and further assisted, by gravitational forces during flexion, due to the trough component tilting downwardly toward its anterior end. It is claimed that during flexion the ball component will travel along the feature described as a "substantially flat portion".

Most recent observations of human spinal movements and the biomechanics involved in relation to flexion and extension indicate that, in a prosthetic spinal disc replacement, there may be a need to provide anteroposterior translation that is controlled or constrained in a way which more closely mimics the natural spinal intervertebral disc motion. Recent tests indicate that it may be beneficial to allow translation in an anterior direction, but in a manner that necessitates exertion from the patient, as opposed to free translation that is promoted by gravitational forces during the downward tilt that results from flexion.

It is also known that in flexion the facet joints of adjacent vertebrae move together at a particular angle of arc. It would therefore be advantageous if the spinal implant was designed to provide a translational motion that was controlled and also ensured that facet joints moved anatomically at the same or similar angle of arc, as would normally be the case in spinal joint movement during natural flexion motion.

As has already been said, the axis of rotation for any two adjacent vertebrae is unique and during flexion or extension will shift, or translate in varying ways and to varying degrees. For example the cervical joints C1/C2 and C2/C3 in flexion, will move and translate in a different way and at a different angle to joints C4/C5, C5/C6 or C6/C7. Similarly, lumbar joints will move in a different direction and at a different angle to cervical joints. Further, lumbar joints L1/L2 will tend to translate at a different angle and direction to lumbar joints L5/S1.

Moreover, whilst it is desirous to provide prosthetic spinal discs that allow translation and rotation it would be more beneficial to provide prosthetic spinal discs that allowed translation in a particular direction whereby the degree or extent of that translation was controlled, depending on the positioning of the prosthesis in the spinal column, the overall aim being to more closely mimic the natural movements of the spine, thereby reducing discomfort and increasing manoeuvrability.

Recent observations also indicate that there may be a need to restrict and control the lateral movement of spinal disc replacement joints so as to more closely resemble the side-to-side motion possible with natural spinal disc tissue. Further observations also indicate that it would be beneficial to the patient if the degree of flexion permitted by the prosthesis was reduced, when flexion is combined with and during rotation.

Several previously-known devices rely on attachment flanges that extend beyond the surfaces of the vertebrae to which the device is attached. This has been found to be undesirable, as the extending flanges may interfere with or injure adjacent tissue. For example, in cervical spinal disc implants, flanges that extend into the immediately adjacent delicate oesophageal area may interfere with swallowing and speech, and may cause perforation and possible infection.

It is also of course advantageous to provide for and encourage bone growth wherever possible, so as to ensure that implants are permanently and securely affixed in place. In the case of lumbar implants, where the inferior faces of the lumbar vertebrae generally have pronounced hollows or concavities, it would be advantageous to reduce the volume of these hollow regions following removal and replacement of the intervertebral disc, so that the gap between the vertebra and the upper prosthesis plate is quickly bridged during bone in growth, ensuring the early build up of bone and the secure retention of the prosthesis.

Many of the examples of prior art are based on experimental devices that suffer disadvantages of one form or another and are not widely accepted. The search for a working spinal disc replacement that accurately reproduces the anatomical movement of a natural intervertebral disc has not yet yielded success. There remains a need for a prosthetic disc which can accurately mimic natural segmental spinal motion, relieve pain, provide durability for long-term or life-time use and which can be easily implanted into a disc space.

The closest prior art is disclosed in US-A-5,888,226.

### SUMMARY OF THE INVENTION

It is an objective of the present invention to overcome some or all of the disadvantages of known prosthetic spinal discs.

According to a first aspect of the present invention there is provided an articulating spinal disc prosthesis, said prosthesis including a first member for engagement with an inferior surface of a first vertebra in use and a second member for engagement with a superior surface of a second vertebra in use, said first member having a vertebra contacting surface and a convex bearing surface provided on an opposite surface thereof, said second member having a vertebra contacting surface and a concave bearing surface provided on an opposite surface thereof, the convex bearing surface of the first member engaging with the concave bearing surface of the second member in use, and wherein the concave bearing surface of the second member includes an inclined ramp.

Preferably the first and/or second members are in the form of plate like members. These plate like members are relatively thin and when engaged together are typically of an equivalent size to a normal disc.

The first and/or second members further include an anterior facing surface, a posterior facing surface, a first lateral surface and a second lateral surface.

Preferably the inclined ramp is provided on the side of the second member nearest to the anterior surface. The inclined ramp, in transverse perpendicular cross section, typically takes the form of a radius.

Preferably the inclined ramp makes the concave bearing surface of the second member, at least in part, asymmetrical about the anterior to posterior axis thereof.

With the first and second members engaged to their respective vertebrae in use, the convex bearing surface engages and cooperates with the concave bearing surface and ramp, thereby permitting rotation and inclined translation with respect to each other. The degree of inclined translation is dictated by the angular presentation of the ramp. The chosen angle of inclination of the ramp will be influenced by the positioning of the prosthetic disc in the spine (i.e. the angle will be preselected to more accurately mimic natural spinal movement applicable to the site of the prosthetic spinal disc insertion).

The ramp is typically linear or planar in form and is provided at an angle different to the angle of the remaining surfaces of the concave portion. The inclination of the ramp is sufficient to allow inclined translation of the second member with respect to the first member in a controlled manner which closely resembles normal movement of the motion segment, particularly taking into account the physiological movement of the facet joints posteriorly.

Thus, in one embodiment, the present invention provides an articulating spinal disc prosthesis which comprises a first upper plate member for engagement with the inferior surface of a first vertebra and a second lower plate member for engagement with the superior surface of a second vertebra, wherein the said upper plate has a vertebra contacting surface, an opposite convex bearing surface, an anterior facing side, a posterior facing side, a first lateral side and a second lateral side. The said lower plate has a vertebra contacting surface, an opposite concave bearing surface, an anterior facing side, a posterior facing side, a first lateral side and a second lateral side. The concave bearing surface of the lower plate includes, on the side nearest to the anterior facing side, an inclined ramp, which, in transverse perpendicular cross-section, takes the form of a radius.

The angle of inclination of the ramp is typically measured in relation to the median plane of the prosthetic joint, the median plane of the joint being substantially parallel to the axial plane.

Preferably the angle of inclination of the ramp falls within the range 5° to 60°, further preferably within the range 10° to 45°, most preferably within the range 15° to 30° and ideally within the range 20° to 25°.

In one embodiment the convex bearing surface of the first member can be in the form of a substantially spherical ball, dome or hemisphere.

Preferably the substantially concave bearing surface of the second member is substantially spherical in shape towards the posterior side or on the side opposite to the inclined ramp. The inclined ramp is typically provided towards the anterior side of the second member when the first and second members are in position and engaging with their respective adjacent vertebra.

In a further embodiment the said convex bearing surface of the first member can be in the form of a substantially aspherical ball, dome or hemisphere. The radius of the ball dome or hemisphere in the coronal and axial planes is typically greater than the radius in the sagittal plane.

Preferably the substantially concave bearing surface of the second member is substantially aspherical in shape towards the posterior side or the side opposite to the inclined ramp. The inclined ramp is typically provided towards the anterior side of the second member, when the first and second members are in position and engaging with their respective adjacent vertebra. The said substantially aspheric bearing surface typically has a radius that is greater in the coronal and axial planes than in the sagittal plane.

Preferably the said vertebra contacting surface of the first member is substantially flat.

In a further embodiment the said vertebra contacting surface of the first member can include a pronounced hemispherical or aspherical dome. The dome can be positioned in a location of the inferior vertebra contacting surface that closely aligns with the pronounced hollows or concavities of the lumbar vertebrae.

It is to be understood that the vertebra contacting surface of the second member can also include a pronounced hemispherical or aspherical dome.

The first and/or second members can also include engaging means for engaging with an insertion tool. The means can be in the form of one or more open slots, apertures and/or channels. Preferably the engaging means are positioned towards the lateral sides of the said vertebra contacting surfaces. Particularly, but not necessarily exclusively, when the engaging means are in the form of open slots or channels, said open slots are positioned so as to permit insertion of a specialised tool, the tool being inserted from above and below the first and second members in a sandwich action.

Thus, the engaging means are typically provided at corresponding and generally parallel locations on the first and second members. The engaging means can be provided across substantially the whole surface of the first and/or second members or on a part thereof.

In a further embodiment, and particularly but not necessarily exclusively when engaging means of the first and second members include one or more holes or apertures, the engaging means are positioned on the anterior sides of the said first and second members and further preferably are displaced towards the lateral sides of the said members. The said holes are positioned so as to permit insertion of a specialised tool, the tool being inserted from the anterior side of the first and second members.

Both the first and/or second members can include attachment means for engaging with their respective adjacent vertebrae.

Preferably the attachment means include one or more pins, spikes, screws, substantially perpendicular fins and/or the like.

Preferably a tool is provided for use in implanting the disc prosthesis according to the present invention. The tool typically includes a handle portion and a prosthesis engaging portion having means to releasably engage or hold one or more parts of the prosthesis.

The prosthesis engaging portion typically engages with the engaging means of the first and/or second members.

Preferably the handle portion is elongate and further preferably the prosthesis engaging portion means include one or more spindles or arms.

The invention also contemplates a tool for use in implanting an intervertebral joint prosthesis, said tool having a handle portion and a prosthesis-engaging portion having elements that hold the one or more parts of the said intervertebral joint prosthesis.

If the tool is used for implanting cervical intervertebral joint prosthesis, the prosthesis-engaging portion consists of elements in the form of slender spindles which engage with engaging means, typically grooves or channels, above and below the said joint prosthesis, provided in the vertebrae contacting surfaces for the purpose of spinal disc implantation.

If the tool is used for implanting a lumbar intervertebral joint prosthesis, the prosthesis-engaging portion typically consists of elements in the form of slender spindles which engage with engaging means, typically in the form of holes in the anterior sides of the said joint prosthesis, provided for the purpose of spinal disc implantation.

The present invention provides an articulating spinal disc prosthesis, and tools for implantation, which allows as near normal a range of articulations between the two adjacent vertebrae, to which the prosthesis has been secured. The advantages of the present invention will become clear to one of ordinary skill in the art, once reference is made to the drawings and the detailed description herein.

### BRIEF DESCCRIPTION OF THE DRAWING

FIG 1 is part-sectional view, showing one embodiment of the prosthesis of the present invention in place between adjacent spinal vertebrae, the view being in the Sagittal plane.
FIG 2 shows a first preferred embodiment of the prosthesis.
FIG2a is a side elevation view; FIG2b is a plan view and FIG 2c is a perspective view of the same prosthesis.
FIG 3a is a side elevation of a first upper plate member of the prosthesis shown in FIG 2;
FIG 3b is an underside view of the same upper plate.
FIG 4 shows a front elevation, plan and a sectioned side elevation of a second lower plate member of the prosthesis shown in FIG 2.
FIG 5 is a side elevation view of a second preferred embodiment of the prosthesis of the present invention.
FIG 6a is side elevation view of the Upper Pate of the prosthesis shown in FIG 5;
FIG 6b is an elevation looking from the front or anterior side and FIG 6c is an elevation looking on the underside of the same Upper Plate.
FIG 7a is a plan view of the Lower Plate of the prosthesis shown in FIGS; FIG 7b is an elevation looking from the front or anterior side; FIG 7c is a sectioned side elevation looking from the front or anterior side; FIG 7c is a sectioned side elevation and FIG 7d is a perspective view of the same Lower Plate.
FIG 8 shows a side elevation, with top and underside views, of the Upper Pate of yet a further embodiment of the prosthesis of the present invention.
FIG 9 shows several views of an insertion tool for use in the insertion of a number of embodiments of the prosthesis of the present invention; FIG 9a being a first side elevation, FIG 9b being a second side elevation, FIG 9c being an end elevation (with prosthetic disc in engagement) and FIG 9d being a perspective view (with prosthetic disc in engagement).
FIG 10 shows several views of an insertion tool for use in the insertion of further embodiments of the prosthesis of the present invention; FIG 10a being a first side elevation, FIG 10b being a second side elevation, FIG 10c being an end elevation (with prosthetic disc in engagement) and FIG 10d being a perspective view (with prosthetic disc in engagement).

### DETAILED DESCRIPTION OF THE INVENTION

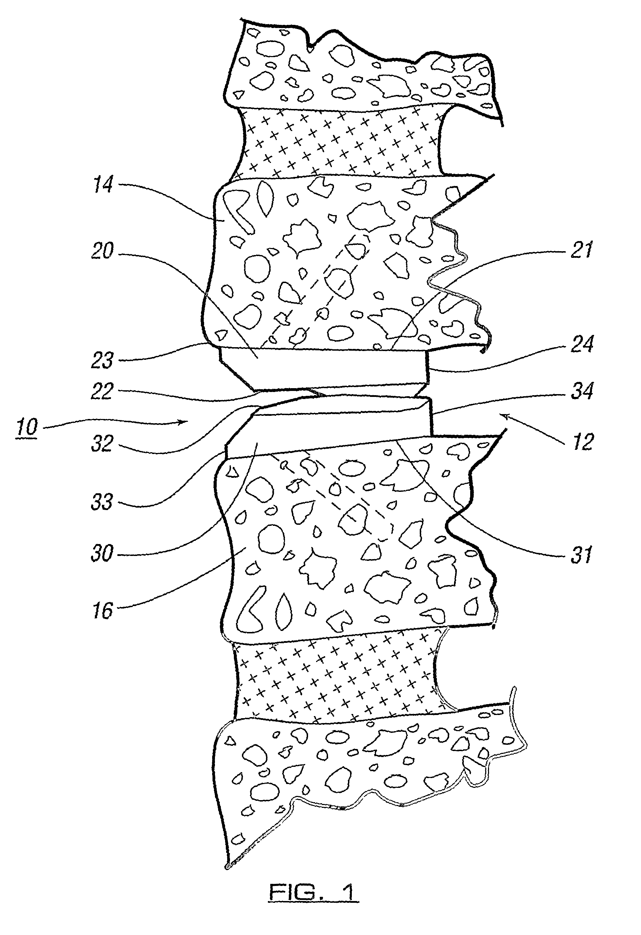

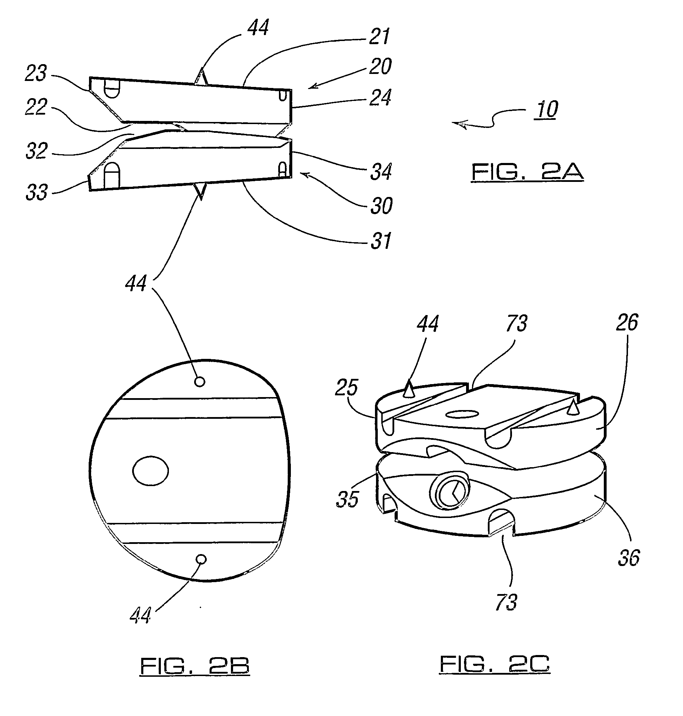

Referring now to FIGS 1 and 2, there is shown one embodiment of the spinal disc prosthesis 10 of the present invention. Prosthesis 10 includes a first upper plate member 20 and a second lower plate member 30. Upper plate 20 includes s vertebra contacting surface 21, an opposite surface 22, and anterior facing side 23, a posterior facing side 24, a first lateral side 25 and a second lateral side 26. Lower plate 30 includes a vertebra contacting surface 31, an opposite surface 32, and anterior facing side 33, a posterior facing side 34, a first lateral side 35 and a second lateral side 36.

In an intervertebral disc space 12 (FIG 1) between two adjacent vertebra 14 and 16 in use, upper plate 20 is fixed to the upper adjacent vertebra 14 and lower plate 30 is fixed to the lower adjacent vertebra 16 so that the two plates 20 & 30 align with an engage each other.

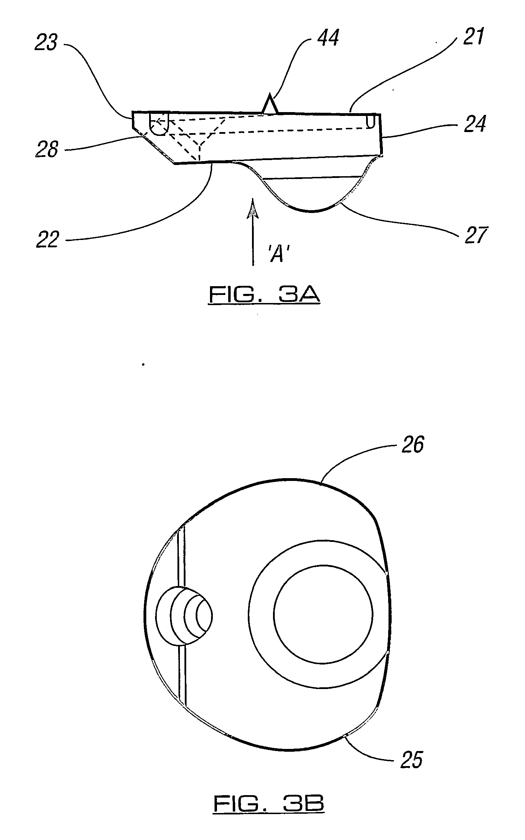

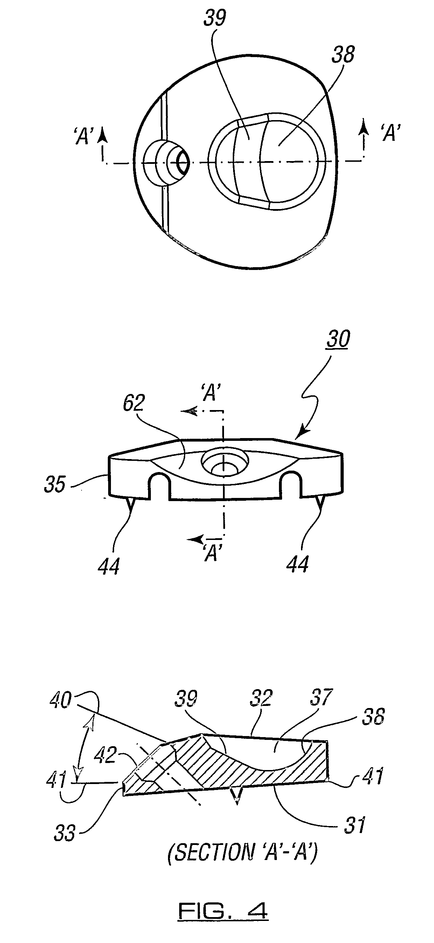

In one embodiment (shown in FIGS 2, 3 & 4), upper plate 20 includes a substantially flat vertebra contacting surface 21 and an opposite surface 22 that includes a substantially convex surface 27 for engagement with lower plate 30. Lower plate 30 includes a substantially flat vertebra contacting surface 31 and an opposite surface 32, which includes a substantially concave surface 37 for engagement with the convex surface 27 of upper plate 20.

The substantially concave surface 37 of lower plate 30 comprises a spherical surface 38 and an inclined ramp 39. The inclined ramp 39 is positioned so that plane 40 of the ramp is at angle alpha, relative to the median plane 41 of the prosthesis. The angle alpha is selected to suit individual patients and is only determined, once the intended position of the prosthesis 10 in the spinal column has been established; but the ramp angle alpha will typically fall within the range 5° to 60°

In a particular embodiment the angled surfaces 28 and 42, on the upper plate 20 and the lower plate 30 respectively, allow the upper and lower plates to be secured to the adjacent vertebrae. This angled surface is typically provided along a peripheral edge of the bearing surfaces of the plates 20, 30 and further preferably adjacent anterior surfaces thereof.

The surfaces 28, 42 provide necessary clearance for the insertion of fixing screws 43 (not shown), as well as clearance for the blade element of a screw driving tool. The fixing screws would be inserted through opening 42 and can be used for initial stabilisation of the prosthesis/implant. Screw 43 could also be a headed pin or a perpendicular keel (perpendicular to the lower and upper plates respectively).

Vertebrae attachment means in the form of projections 44 assist in retaining the prosthesis in position in the intervertebral disc space 12. Projections 44 engage with adjacent vertebrae.

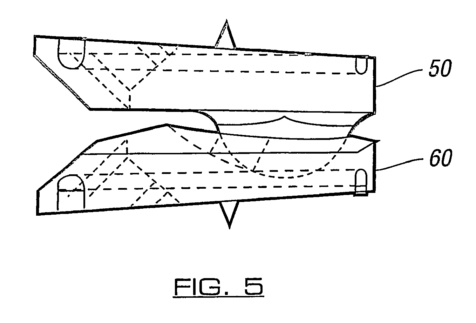

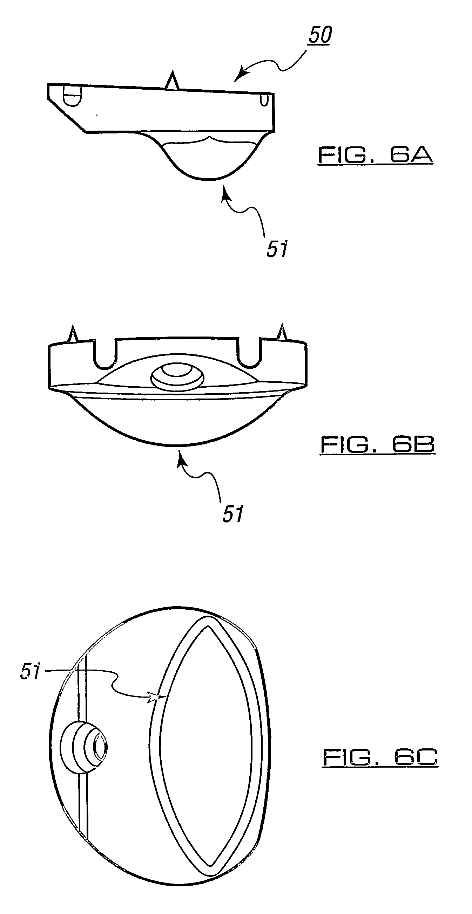

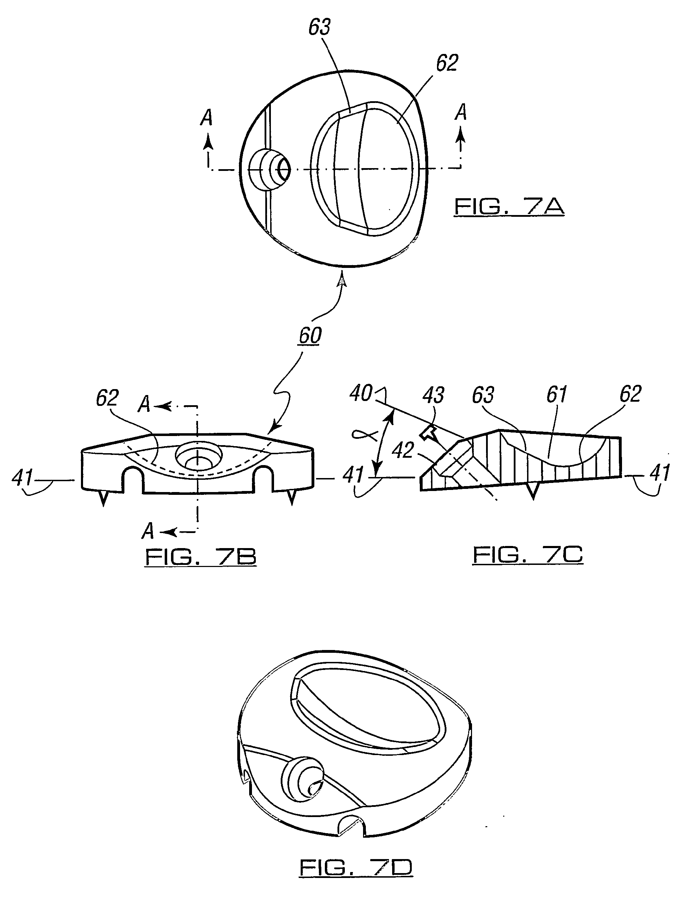

In a further embodiment, shown in FIGS 5 to 7, the upper plate 50 includes a convex surface 51 that is substantially aspherical in shape and the lower plate 60 includes a concave surface 61 that comprises a substantially aspherical surface 62 and an inclined ramp 63. The geometry of the aspherical surfaces 51 & 62 can be more readily appreciated by reference to accompanying FIG 6 wherein the radius of the said substantially aspherical surface 51 is greater in the Coronal plane (FIG 6b) and also in the Axial plane (FIG 6c) than is the case in the Sagittal plane (FIG 6a); similarly the radius of the said aspherical surface 62 (shown in FIG 7) is greater in the Coronal plane (FIG 7b) and also in the axial plane (FIG 7a) than is the case in the Sagittal plane (FIG 7c).

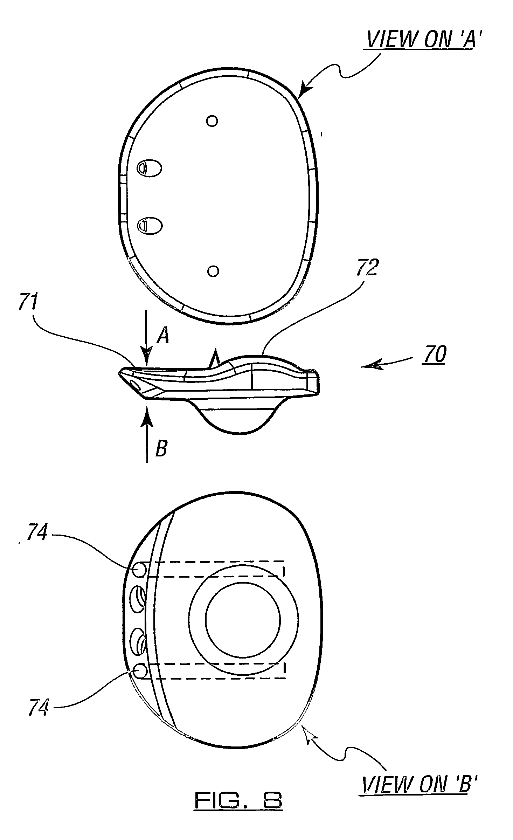

In yet a further embodiment, shown in FIG 8, the upper plate 70 includes a vertebra contacting surface 71 and a pronounced dome 72, the dome 72 being positioned in a location of the vertebra contacting surface 71 so as to align with the main hollow in the inferior surface of a normally developed lumbar vertebra. The position of this dome may vary in the anterior-posterior plane.

In a further embodiment the upper plate 20 and lower pate 30 of the prosthesis are provided with engaging means in the form of open slots or channels 73 (FIG 2) in the vertebrae contacting surfaces to facilitate the engagement of prosthesis engaging elements of an insertion tool during the process of insertion of the prosthesis into the spinal column.

In a further embodiment engaging means in the form of holes 74 (FIG 8) are provided in the anterior side of the upper and lower plates of the prosthesis, the holes being used to facilitate the engagement of prosthesis engaging elements of an insertion tool during the process of insertion of the prosthesis into the spinal column.

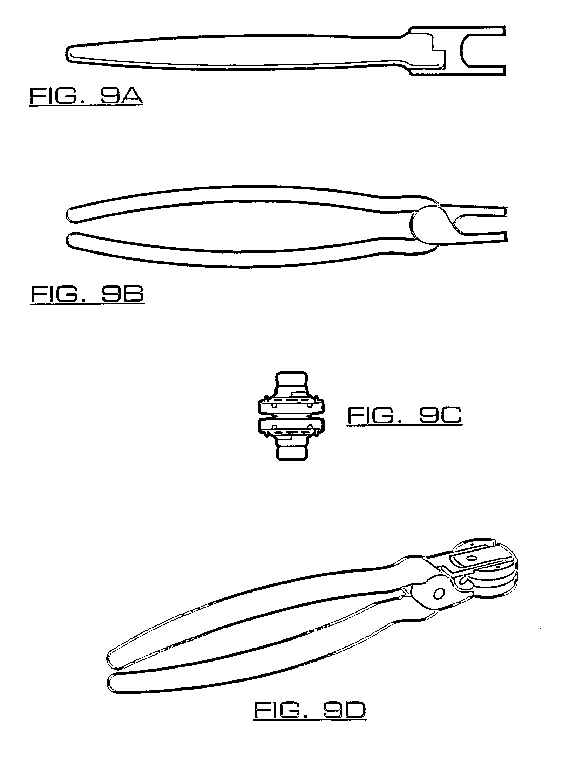

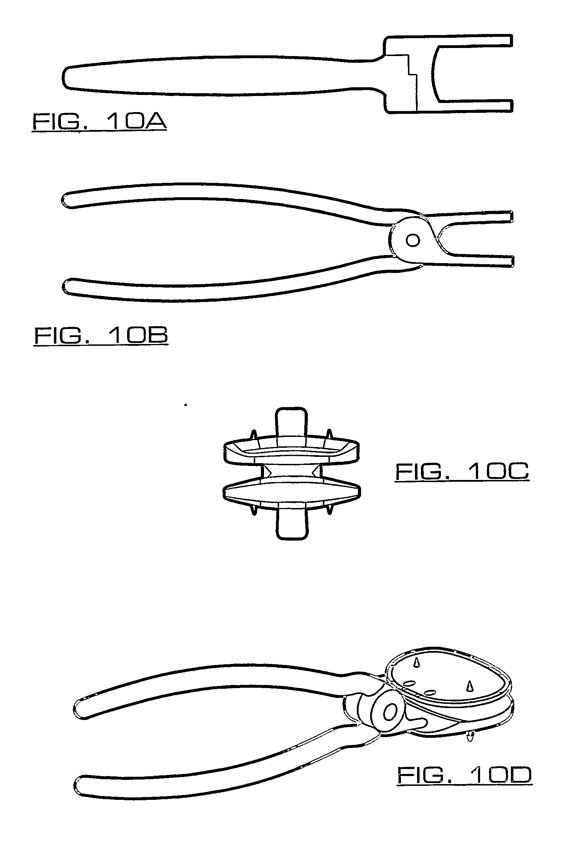

Tools used for insertion of prosthetic spinal discs are shown at FIGS 9 & 10.

FIG 9 shows several views of a tool for use during the insertion of a spinal disc prosthesis into the cervical region of the spine. The tool is movable between open and closed positions and in the closed position, prosthesis engaging portions are located above and below the two-part prosthesis, engaging into the slots provided in the vertebrae contacting surfaces of the upper and lower plates of the implant.

FIG 10 shows several views of a tool for use during the insertion of a two-part spinal disc prosthesis into the lumbar region of the spine. The disc-engaging elements of the tool are inserted into holes provided in the anterior facing sides of the upper and lower plates that comprise the implant.

The prosthesis engaging portions include arm members or spindles in either embodiment.

It will be appreciated by persons skilled in the art that any of the features described hereinabove can be provided alone or in combination with one or more other features in accordance with the present invention.

## Claims

1. An articulating spinal disc prosthesis (10), said prosthesis including a first member (20, 50, 70) for engagement with an inferior surface of a first vertebra (14) in use and a second member (30, 60) for engagement with a superior surface of a second vertebra (16) in use, said first member (20, 50, 70) having a vertebra contacting surface (21, 71) and a convex bearing surface (27, 51, 72) provided on an opposite surface (22) thereof, said second member (30, 60) having a vertebra contacting surface (31) and a concave bearing surface (37, 62) provided on an opposite surface thereof (32), the convex bearing surface (27, 51, 72) of the first member (20, 50, 70) engaging with the concave bearing surface (37, 62) of the second member (30, 60) in use, **characterised in that** the concave bearing surface (37, 62) of the second member (30, 60) includes an inclined planar ramp (39, 63).

2. A prosthesis according to claim 1 wherein the first (20, 50, 70) and/or second (30, 60) members are in the form of plate members.

3. A prosthesis according to claim 1 wherein the first (20, 50, 70) and/or second (30, 60) members further include an anterior facing surface (23, 33), a posterior facing surface (24, 34), a first lateral surface (25, 35) and a second lateral surface (26, 36).

4. A prosthesis according to claim 3 wherein the inclined ramp (39, 63) is provided on the side of the second member (30, 60) nearest to the anterior surface (33).

5. A prosthesis according to claim 1 wherein the inclined ramp (39, 63), in transverse perpendicular cross section, takes the form of a radius.

6. A prosthesis according to claim 3 wherein the inclined ramp (39, 63) makes the concave bearing surface (37, 62) of the second member (30, 60), at least in part, asymmetrical about the anterior to posterior axis thereof.

7. A prosthesis according to claim 1 wherein the angle of inclination of the ramp (39, 63) is in the range of 5° to 60°.

8. A prosthesis according to claim 1 wherein the angle of inclination of the ramp (39, 63) is in the range of 10° to 45°.

9. A prosthesis according to claim 1 wherein the angle of inclination of the ramp (39, 63) is in the range of 15° to 30°.

10. A prosthesis according to claim 1 wherein the angle of inclination of the ramp (39, 63) is in the range of 20° to 25°.

11. A prosthesis according to claim 1 wherein the convex bearing surface (27, 51) of the first member (20, 50, 70) is in the form of a substantially spherical ball, dome and/or hemisphere.

12. A prosthesis according to claim 1 wherein the concave bearing surface (37, 62) of the second member (30, 60) is substantially spherical (38) on a side opposite to the side on which the inclined ramp (39, 63) is provided.

13. A prosthesis according to claim 1 wherein the concave bearing surface (37, 62) of the second member (30, 60) is substantially spherical (38) towards a posterior side (34) thereof.

14. A prosthesis according to claim 1 wherein the convex bearing surface (27, 51, 72) of the first member (20, 50, 70) is in the form of a substantially aspherical ball, dome or hemisphere.

15. A prosthesis according to claim 14 wherein the radius of the ball, dome or hemisphere in the coronal and axial planes is greater than the radius of the same in the sagittal plane.

16. A prosthesis according to claim 1 wherein the concave bearing surface (37, 62) of the second member (30, 60) is substantially aspherical in shape on a side opposite to the side on which the inclined ramp (39, 63) is provided.

17. A prosthesis according to claim 1 wherein the concave bearing surface (37, 62) of the second member (30, 60) is substantially aspherical in shape towards a posterior side (34) thereof.

18. A prosthesis according to claim 17 wherein the aspheric bearing surface has a radius that is greater in the coronal and axial planes than in the sagittal plane.

19. A prosthesis according to claim 1 wherein the vertebrae contacting surface (21, 71, 31) of the first (20, 50, 70) and/or second member (30, 60) is substantially flat.

20. A prosthesis according to claim 1 wherein the vertebrae contacting surface (21, 71) of the first member (20, 50, 70) includes a hemispherical or aspherical dome portion (72).

21. A prosthesis according to claim 20 wherein the hemispherical dome portion (72) is located on an inferior part of the vertebrae contacting surface (21, 71) in use.

22. A prosthesis according to claim 1 wherein the first (20, 50, 70) and/or second (30, 60) members include engaging means for engaging with an insertion tool.

23. A prosthesis according to claim 22 wherein the engaging means include one or more channels, slots and/or apertures (74).

24. A prosthesis according to claim 22 wherein the engaging means are positioned towards the lateral sides of the vertebrae contacting surfaces (21, 71, 31).

25. A prosthesis according to claim 22 wherein the engaging means are positioned on the anterior sides of the members (20, 50, 70, 30, 60).

26. A prosthesis according to claim 1 wherein the first (20, 50, 70) and/or second (30, 60) members include attachment means for engaging with adjacent vertebrae (14, 16) in use.

27. A prosthesis according to claim 26 wherein the attachment means includes any or any combination of one or more pins, spikes, screws or substantially perpendicular fins.

28. A prosthesis according to claim 1 wherein at least a portion of the first (20, 50, 70) and/or second (30, 60) members is angled towards an outer peripheral edge thereof.

29. A prosthesis according to claim 1 wherein the concave bearing surface (37, 62) is different in shape to the convex bearing surface (27, 51).

## Patentansprüche

1. Eine gelenkige Wirbelscheibenprothese (10), wobei die genannte Prothese ein erstes Element (20, 50, 70) zum Eingriff in eine untere Fläche eines eingesetzten ersten Wirbels (14) aufweist und ein zweites Element (30, 60) zum Eingriff in eine Oberfläche eines zweiten eingesetzten Wirbels (16), wobei das besagte erste Element (20, 50, 70) eine Wirbelkontaktfläche (21, 71) und eine konvexe Lagerfläche (27, 51, 72) auf einer gegenüberliegenden Oberfläche (22) davon aufweist, wobei das zweite Element (30, 60) eine Wirbellkontaktfläche (31) und eine konkave Lagerfläche (37, 62) auf einer gegenüberliegenden Oberfläche derselben (32) aufweist, wobei die konvexe Lagerfläche (27, 51, 72) des ersten Elements (20, 50, 70) in die konkave Lagerfläche (37, 62) des zweiten eingesetzten Elements (30, 60) eingreift, **dadurch gekennzeichnet, dass** die konkave Lagerfläche (37, 62) des zweiten Elements (30, 60) eine schräge Rampe (39, 63) aufweist.

2. Eine Prothese gemäß Anspruch 1, wobei die ersten Elemente (20, 50, 70) bzw. zweiten Elemente (30, 60) plattenförmige Elemente sind.

3. Eine Prothese gemäß Anspruch 1, wobei die ersten Elemente (20, 50, 70) bzw. zweiten Elemente (30, 60) zudem eine anteriore Oberfläche (23, 33), eine posteriore Oberfläche (24, 34), eine erste laterale Oberfläche (25, 35) und eine zweite laterale Oberfläche (26, 36) aufweisen.

4. Eine Prothese gemäß Anspruch 3, wobei die schräge Rampe (39, 63) auf der der anterioren Oberfläche (33) am nächsten gelegenen Seite des zweiten Elements (30, 60) angeordnet ist.

5. Eine Prothese gemäß Anspruch 1, wobei die schräge Rampe (39, 63) in ihrem diagonalen senkrechten Querschnitt die Form eines Radiuses hat.

6. Eine Prothese gemäß Anspruch 3, wobei die schräge Rampe (39, 63) die konkave Lagerfläche (37, 62) des zweiten Elements (30, 60) zumindestens teilweise asymmetrisch um die anteriore zur posterioren Achse davon verläuft.

7. Eine Prothese gemäß Anspruch 1, wobei der Neigungswinkel der Rampe (39, 63) zwischen 5° und 60 ° liegt.

8. Eine Prothese gemäß Anspruch 1, wobei der Neigungswinkel der Rampe (39, 63) zwischen 10° und 45 ° liegt.

9. Eine Prothese gemäß Anspruch 1, wobei der Neigungswinkel der Rampe (39, 63) zwischen 15° und 30 ° liegt.

10. Eine Prothese gemäß Anspruch 1, wobei der Neigungswinkel der Rampe (39, 63) zwischen 20° und 25º liegt.

11. Eine Prothese gemäß Anspruch 1, wobei die konvexe Lagerfläche (27, 51) eines ersten Elements (20, 50, 70) die Form einer hauptsächlich rundförmigen Kugel, Kuppel oder Halbkugel hat.

12. Eine Prothese gemäß Anspruch 1, wobei die konkave Lagerfläche (37, 62) des zweiten Elements (30, 60) eine hauptsächliche kugelförmige (38) Form auf der der schrägen Rampe (39, 63) gegenüberliegenden Seite aufweist

13. Eine Prothese gemäß Anspruch 1, wobei die konkave Lagerfläche (37, 62) des zweiten Elements (30, 60) eine hauptsächliche kugelförmige (38) Form in der Nähe der posterioren Seite (34) davon aufweist.

14. Eine Prothese gemäß Anspruch 1, wobei die konvexe Lagerfläche (27, 51, 72) des ersten Elements (20, 50, 70) die Form einer hauptsächlich asphärischen Kugel, Kuppel oder Halbkugel aufweist.

15. Eine Prothese gemäß Anspruch 14, wobei der Radius der rundförmigen Kugel, Kuppel oder Halbkugel in den koronaren und axialen Ebenen größer als der Radius auf der sagittalen Ebene ist.

16. Eine Prothese gemäß Anspruch 1, wobei die konkave Lagerfläche (37, 62) des zweiten Elements (30, 60) eine hauptsächliche kugelförmige Form auf der der schrägen Rampe (39, 63) gegentiberliegenden Seite aufweist.

17. Eine Prothese gemäß Anspruch 1, wobei die konkave Lagerfläche (37, 62) des zweiten Elements (30, 60) eine hauptsächliche kugelförmige Form in der Nähe der posterioren Seite (34) davon aufweist.

18. Eine Prothese gemäß Anspruch 17, wobei die asphärische Lagerfläche einen Radius aufweist, der größer auf den koronaren und axialen Ebenen als auf der sagittalen Ebene ist.

19. Eine Prothese gemäß Anspruch 1, wobei die Wirbelkontaktfläche (21,71, 31) des ersten (20, 50, 70) bzw. zweiten (30, 60) Elements im Wesentlichen eben ist.

20. Eine Prothese gemäß Anspruch 1, wobei die Wirbelkontaktfläche (21, 71) des ersten Elements (20, 50, 70) einen Halbkugel- oder runden Kuppelteil (72) beinhaltet.

21. Eine Prothese gemäß Anspruch 20, wobei der Halbkugelteil (72) an einer Stelle der unteren Wirbelkontalctfläclie (21, 71) angeordnet ist.

22. Eine Prothese gemäß Anspruch 1, wobei die ersten (20, 50, 70) bzw. zweiten (30,60) Elemente eine Einsteckvorrichtung zum Eingriff mit einem Einsteckwerkzeug aufweisen.

23. Eine Prothese gemäß Anspruch 22, wobei die Einsteckvorriehtungen einen oder mehrere Kanäle, Schlitze oder Öffnungen (74) aufweisen.

24. Eine Prothese gemäß Anspruch 22, wobei die Einsteckvorrichtungen in der Nähe der lateralen Seiten der Wirbelkontaktflächen (21, 71, 31) angeordnet sind.

25. Eine Prothese gemäß Anspruch 22, wobei die die Einsteckvorrichtungen auf den anterioren Seiten der Elemente (20, 50, 70, 30, 60) angeordnet sind.

26. Eine Prothese gemäß Anspruch 1, wobei die ersten (20, 50, 70) bzw. zweiten (30, 60) Elemente Befestigungsvorrichtung zur Befestigung an ihre nebenliegende Wirbel (14,16) aufweisen.

27. Eine Prothese gemäß Anspruch 26, wobei die Befestigungsvorrichtung ein oder jegliche Kombination eines oder mehrerer Stifte, Nägel, Schrauben oder im Wesentlichen senkrechter Rippen bzw. dergleichen aufweist.

28. Eine Prothese gemäß Anspruch 1, wobei mindestens ein Teil der ersten (20, 50, 70) bzw. zweiten (30, 60) Elemente einem äußeren periphären Rand davon gegenüber angewinkelt sind.

29. Eine Prothese gemäß Anspruch 1 wobei sich die Form der konkaven Lagerfläche (37, 62) von der Form der konvexen Lagerfläche (27, 51) unterscheidet.

## Revendications

1. Prothèse articulée de disque intervertébral (10), ladite prothèse comprenant un premier élément (20, 50, 70) s'engageant sur la surface inférieure d'une première vertèbre (14) durant l'utilisation et un second élément (30, 60) s'engageant sur la surface supérieure d'une deuxième vertèbre (16) durant l'utilisation, ledit premier élément (20, 50, 70) ayant une surface en contact avec la vertèbre (21, 71) et une surface d'appui convexe (27, 51, 72) réalisée sur la surface opposée (22) de celle-ci, ledit second élément (30, 60) ayant une surface en contact avec la vertèbre (31) et une surface d'appui concave (37, 62) réalisée sur la surface opposée de celle-ci (32), la surface d'appui convexe (27, 51, 72) du premier élément (20, 50, 70) s'engageant dans la surface d'appui concave (37, 62) du deuxième élément (30, 60) durant l'utilisation, **caractérisée en ce que** la surface d'appui concave (37, 62) du deuxième élément (30, 60) comprend une rampe en plan incliné (39, 63).

2. Prothèse selon la revendication 1, le premier élément (20, 50, 70) et/ou le second élément (30, 60) ayant la forme d'éléments plaques.

3. Prothèse selon la revendication 1, le premier élément (20, 50, 70) et/ou le second élément (30, 60) comprenant en outre une surface face antérieure ((23, 33), une surface face postérieure ((24, 34), une première surface latérale ((25, 35) et une seconde surface latérale (26, 36).

4. Prothèse selon la revendication 3, la rampe inclinée (39, 63) étant réalisée sur le côté du second élément (30, 60) le plus proche de la surface antérieure (33).

5. Prothèse selon la revendication 1, la rampe inclinée (39, 63), en coupe transversale perpendiculaire ayant la forme d'un rayon.

6. Prothèse selon la revendication 3, la rampe inclinée (39, 63) rendant au moins partiellement asymétrique la surface d'appui concave (37, 62) du second élément (30, 60) autour de l'axe antéro-postérieur de celui-ci.

7. Prothèse selon la revendication 1, l'angle d'inclinaison de la rampe (39, 63) étant dans la plage 5 à 60°.

8. Prothèse selon la revendication 1, l'angle d'inclinaison de la rampe (39, 63) étant dans la plage 10 à 45°.

9. Prothèse selon la revendication 1, l'angle d'inclinaison de la rampe (39, 63) étant dans la plage 15 à 30°.

10. Prothèse selon la revendication 1, l'angle d'inclinaison de la rampe (39, 63) étant dans la plage 20 à 25°.

11. Prothèse selon la revendication 1, la surface d'appui convexe (27, 51) du premier élément (20, 50, 70) ayant la forme d'une boule sensiblement sphérique, d'un dôme ct/ou d'un hémisphère.

12. Prothèse selon la revendication 1, la surface d'appui concave (37, 62) du second élément (30, 60) étant sensiblement sphérique (38) du côté opposé à celui sur le quel se trouve la rampe inclinée (39, 63).

13. Prothèse selon la revendication 1, la surface d'appui concave (37, 62) du second élément (30, 60) étant sensiblement sphérique (38) vers le côté postérieur (34) de celui-ci.

14. Prothèse selon la revendication 1, la surface d'appui convexe (27, 51, 72) du premier élément (20, 50, 70) ayant la forme d'une boule sensiblement sphérique, d'un dôme et/ou d'un hémisphère.

15. Prothèse selon la revendication 14, le rayon de la boule, du dôme et/ou de l'hémisphère dans les plans frontal et axial étant supérieur à leur rayon dans le plan sagittal.

16. Prothèse selon la revendication 1, la surface d'appui concave (37, 62) du second élément (30, 60) étant de forme sensiblement sphérique du côté opposé à celui sur lequel se trouve la rampe inclinée (39, 63).

17. Prothèse selon la revendication 1, la surface d'appui concave (37, 62) du second élément (30, 60) étant de forme sensiblement asphérique vers l'extrémité postérieure (34) de celui-ci.

18. Prothèse selon la revendication 17, la surface d'appui asphérique ayant un rayon plus important dans le plan frontal et axial que dans le plan sagittal.

19. Prothèse selon la revendication 1, la surface en contact avec la vertèbre (21, 71, 31) du premier (20, 50, 70) et/ou du second élément (30, 60) étant sensiblement plate.

20. Prothèse selon la revendication 1, la surface en contact avec la vertèbre (21, 71) du premier élément (20, 50, 70) comportant une partie en dôme hémisphérique ou asphérique (72).

21. Prothèse selon la revendication 20, la partie en dôme hémisphérique (72) étant située à la partie inférieure de la surface en contact avec les vertèbres (21, 71) durant l'utilisation.

22. Prothèse selon la revendication 1, le premier (20, 50, 70) et/ou le second élément (30, 60) comprenant des moyens d'engagement pour engagement avec un outil d'insertion.

23. Prothèse selon la revendication 22, les moyens d'engagement comprenant un ou plusieurs évidements, fentes et/ou ouvertures (74).

24. Prothèse selon la revendication 22, les moyens d'engagement étant positionnés vers les côtés latéraux des surfaces en contact avec les vertèbres (21, 71, 31).

25. Prothèse selon la revendication 22, les moyens d'engagement étant positionné sur les côtés antérieurs des éléments (20, 50, 70, 30, 60).

26. Prothèse selon la revendication 1, le premier (20, 50, 70) et/ou le second élément (30, 60) comprenant un moyen de fixation pour les engager avec les vertèbres adjacentes (14, 16) durant l'utilisation.

27. Prothèse selon la revendication 26, le moyen de fixation comprenant un quelconque ou une quelconque combinaison d'une ou de plusieurs broches, pointes, vis ou ailettes sensiblement perpendiculaires.

28. Prothèse selon la revendication 1, au moins une partie du premier (20, 50, 70) et/ou du second élément (30, 60) étant inclinée vers un rebord périphérique extérieur de ceux-ci.

29. Prothèse selon la revendication 1, la surface d'appui concave (37, 62) ayant une forme différente de la surface d'appui convexe (27, 51
